(19) Europäisches Patentamt

European Patent Office

Office européen des brevets

(11) EP 3 432 173 A1

(12) **EUROPEAN PATENT APPLICATION**

(43) Date of publication:
23.01.2019 Bulletin 2019/04

(51) Int Cl.:
*G06F 19/00* (2019.01)    *G16H 50/20* (2018.01)

(21) Application number: 17181838.8

(22) Date of filing: 18.07.2017

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(71) Applicant: **Siemens Aktiengesellschaft**
80333 München (DE)

(72) Inventors:
• **Yang, Yinchong**
81673 München (DE)
• **Tresp, Volker**
81369 München (DE)

(54) **METHOD AND SYSTEM FOR PREDICTIVE CLINICAL DECISION SUPPORT**

(57)    A clinical decision support system (1) comprising a recurrent neural network, RNN, encoder (2) adapted to encode a time series of medical events related to a patient to generate a latent medical history hidden vector, h, representing the medical history of the patient; and a processing unit (3) adapted to calculate therapy recommendations for a user based on the latent medical history hidden vector, h, using a predefined hierarchical target regression model, HTRM, stored in a memory.

## FIG 1

**Description**

The invention relates to a method and a system for providing a predictive clinical decision support for a user, in particular a physician or a nurse.

A user such as a physician has to decide which therapy is most suited for a patient. A patient normally has a medical history including therapies performed in the past. Conventionally, physicians decide which therapy to apply based on their medical experience. A physician may discuss a case with another physician to get support for his clinical decision how to treat a patient. However, the other physicians might not have more experience or might not be available.

Accordingly, it is an object of the present invention to provide a method and system for supporting a user in making clinical decisions and to increase the probability that a suitable therapy is applied to a patient.

This object is achieved according to a first aspect of the present invention by a method having the features of claim 1.

The invention provides according to the first aspect a method for providing a predictive clinical decision support comprising the steps of:

encoding a time series of medical events related to a patient by a recurrent neural network, RNN, encoder to generate a latent medical history hidden vector representing the medical history of the patient and

calculating therapy recommendations for a user by a processor based on the generated latent medical history hidden vector of the patient using a predetermined hierarchical target regression model stored in a memory.

In a possible embodiment of the method according to the first aspect of the present invention, the generated latent medical vector is extended with a latent patient information hidden vector representing static patient background information of the patient to generate a hidden patient profile vector.

In a further possible embodiment of the method according to the first aspect of the present invention, the generated latent medical history hidden vector and the latent patient background information hidden vector are concatenated to provide the patient profile vector supplied to the hierarchical target regression model for calculating therapy recommendations concerning the patient output to the user via an interface.

In a still further possible embodiment of the method according to the first aspect of the present invention, distance metrics are calculated between the patient profile vector of the respective patient and patient profile vectors of other patients to quantify similarities between the patient and other patients and to provide a list of similar patients ranked according to the calculated distance metrics.

In a still further possible embodiment of the method according to the first aspect of the present invention, the time series of medical events comprising binary event vectors representing features of medical events related to the patient is weighted by the recurrent neural network, RNN, encoder to calculate the current latent medical history hidden vector of the patient representing the medical history of the patient.

In a still further possible embodiment of the method according to the first aspect of the present invention, a static background information vector of the patient is mapped by a trained weight mapping matrix to calculate the latent patient information hidden vector of the patient.

In a still further possible embodiment of the method according to the first aspect of the present invention, the hierarchical target regression model comprises several hierarchical levels for calculating conditional probabilities.

In a still further possible embodiment of the method according to the first aspect of the present invention, a category probability is computed that the recommended therapy does belong to a therapy category of a predefined set of therapy categories.

In a still further possible embodiment of the method according to the first aspect of the present invention, marginal probabilities for therapy recommendations are calculated using the hierarchical target regression model on the basis of the hidden patient profile vector and the computed category probability.

In a still further possible embodiment of the method according to the first aspect of the present invention, therapy recommendations are sorted according to their marginal properties to provide a ranking list of possible therapies output to the user to select a therapy for the patient from the ranking list of possible therapies.

In a still further possible embodiment of the method according to the first aspect of the present invention, the therapy with the highest marginal probability is automatically selected and output as the recommended therapy to the user.

In a still further possible embodiment of the method according to the first aspect of the present invention, a machine learning model comprising the hierarchical target regression model, the recurrent neural network, RNN, encoder and the mapping matrix is trained on historical patient data stored in a database.

In a still further possible embodiment of the method according to the first aspect of the present invention, a deviation alert is generated if the therapy selected by the user differs from the therapy having the highest calculated marginal probability.

In a still further possible embodiment of the method according to the first aspect of the present invention, medical actuators attached to the patient and/or located in the vicinity of the patient are automatically controlled in response to the selected therapy.

The invention further provides according to a further aspect a clinical decision support system comprising the features of claim 15.

The invention provides according to the second aspect a clinical decision support system comprising:

a recurrent neural network, RNN, encoder adapted to encode a time series of medical events related to a patient to generate a latent medical history hidden vector representing the medical history of the patient and

a processing unit adapted to calculate therapy recommendations for a user based on the latent medical history hidden vector using a predefined hierarchical target regression model stored in a memory.

In the following, possible embodiments of the different aspects of the present invention are described in more detail with reference to the enclosed figures.

Fig. 1    shows a block diagram of a possible exemplary embodiment of a clinical decision support system according to an aspect of the present invention;

Fig. 2    shows a further block diagram of a possible embodiment of the clinical decision support system illustrated in Fig. 1;

Fig. 3    shows schematically an exemplary use case of the method and system according to the present invention;

Fig. 4    shows a flowchart of a possible exemplary embodiment of a method for providing a predictive clinical decision support according to an aspect of the present invention;

Fig. 5    shows a schematic diagram for illustrating a possible exemplary implementation of a method and system according to the present invention.

Fig. 6    shows a diagram for illustrating the calculation of probabilities as performed by a method and system according to the present invention.

Fig. 7    shows a diagram for illustrating a further possible embodiment of a method and system according to the present invention.

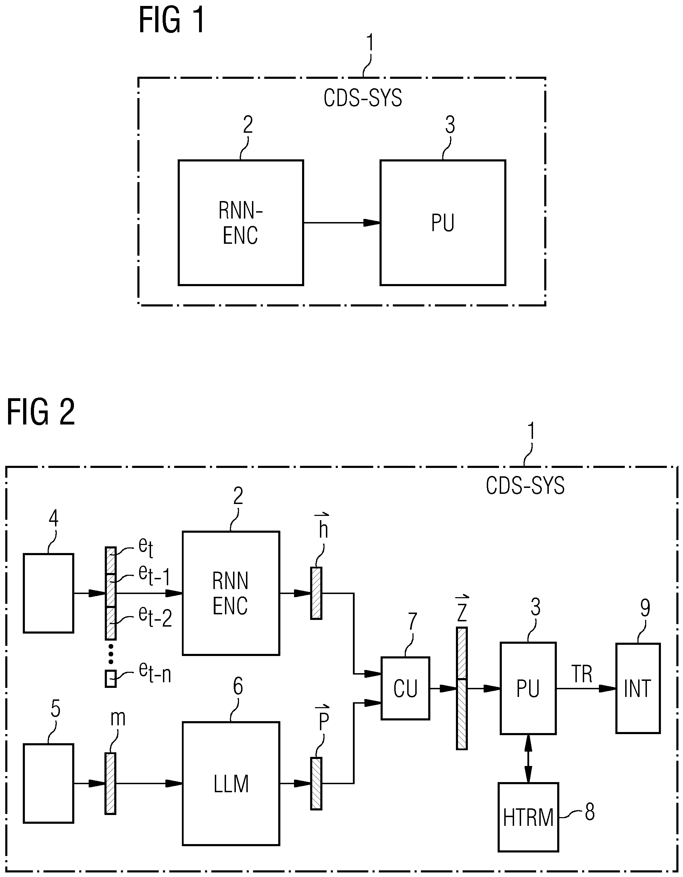

As can be seen in the block diagram of Fig. 1, a clinical decision support system 1 according to the present invention can comprise at least two main components, i.e. a recurrent neural network, RNN, encoder 2 and a processing unit 3 used as a hierarchical response decoder. The recurrent neural network, RNN, encoder 2 is adapted to encode a time series of medical events related to a patient P to generate a latent medical history hidden vector h representing the medical history of the patient. The RNN encoder 2 can in a possible embodiment comprise a LSTM (Long Short-Term Memory) RNN encoder or a Gated Recurrent Unit, GRU-RNN encoder. A time series of medical events related to a patient can be read by the RNN encoder 2 from a database or received via a data interface. The processing unit 3 of the clinical decision support system 1 is adapted to calculate therapy recommendations T-REC for a user U such as a physician based on the latent medical history hidden vector h provided by the RNN encoder 2 using a predefined hierarchical target regression model HTRM stored in a memory. The calculated therapy recommendations T-REC can be output to the user via a user interface of the clinical decision support system 1.

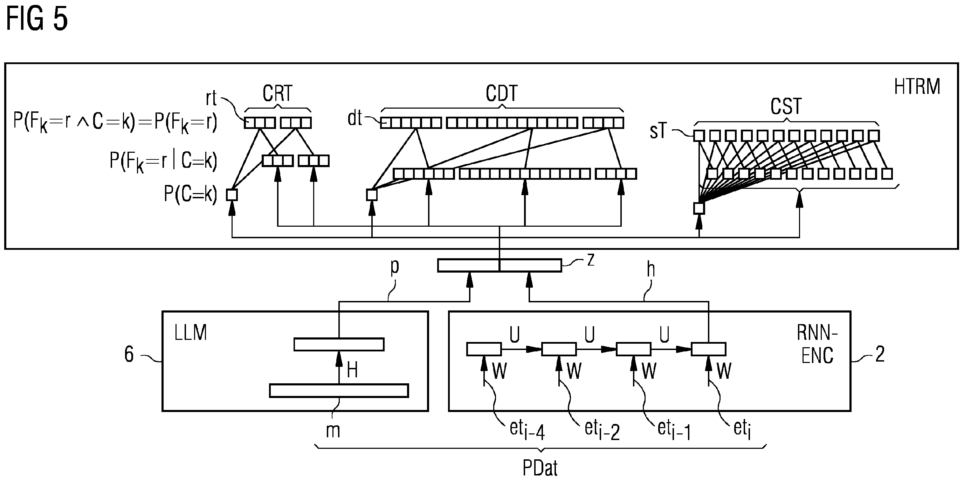

Fig. 2 shows a more detailed block diagram of a possible exemplary embodiment of a predictive clinical decision support system 1 according to an aspect of the present invention. The time series of medical events related to a patient P can comprise binary event vectors e representing features of medical events related to the patient. These binary event vectors e can be derived from a feature vector or a dataset of the patient P. This feature vector can comprise diagnostic results concerning the patient. For instance, the patient may have developed a local rezidiv of a tumor. Further, the feature vector may comprise a staging, i.e. a staging from 0 to 9, a time of the diagnosis and a location where the symptom has occurred in the body of the patient. For instance, the location may indicate in which organ of the patient the local rezidiv has been developed. The different features can be binary encoded, for instance each organ of the body gets a value of 0 or 1 depending whether the respective local rezidiv of the tumor has developed in this organ or not. Accordingly, the features of the patient P concerning medical events reflecting a medical history of the patient at different times t can be preprocessed or encoded to generate binary event vectors e at different time steps t as also illustrated in Fig. 5. The provided binary event vectors e can be loaded from a local memory 4 or received from a remote database via an input interface. In a possible embodiment the event vectors can also be generated and directly supplied to the clinical decision support system 1.

Further, each patient can comprise a dataset of different static features forming static patient background information of the patient P. This static patient dataset can comprise for instance the age of the patient, the height of the patient, the weight of the patient or family medical records and other static patient features. The static patient background information can also be encoded into a static background information vector m as illustrated in Fig. 2. The static background information vector m can be read from a local memory 5 or received from a remote database via an input interface. The time series of medical events comprising a set of binary event vectors e at different time steps t representing features of medical events related to the patient P can be weighted or encoded by the recurrent neural network, RNN, encoder 2 to calculate a current latent medical history hidden vector h of the patient P as shown in Fig. 2. The current latent medical history hidden vector h represents the medical history of the respective patient. In the embodiment illustrated in Fig. 2, the static background information vector m of the patient is mapped by a log linear mapping unit 6 to calculate the latent patient information hidden vector p of the patient as shown in Fig. 2. The latent medical history hidden vector h provided by the RNN encoder 2 is extended with the latent patient information hidden vector p to generate a hidden patient profile vector z as shown in Fig. 2. In a possible embodiment, the generated latent medical history hidden vector h and the latent patient background information hidden vector p are concatenated by a concatenation unit 7 of the predictive clinical decision support system 1 to provide the patient's profile vector z supplied to the processing unit 3 which has access to a hierarchical target regression model HTRM stored in a memory 8. An example of a possible embodiment of a hierarchical target regression model HTRM is illustrated in Fig. 5. The processing unit 3 supplies the calculated hidden patient profile vector z to the hierarchical target regression model HTRM for calculating therapy recommendations concerning the patient which can be output to a user via a user interface 9 of the predictive clinical decision support system 1.

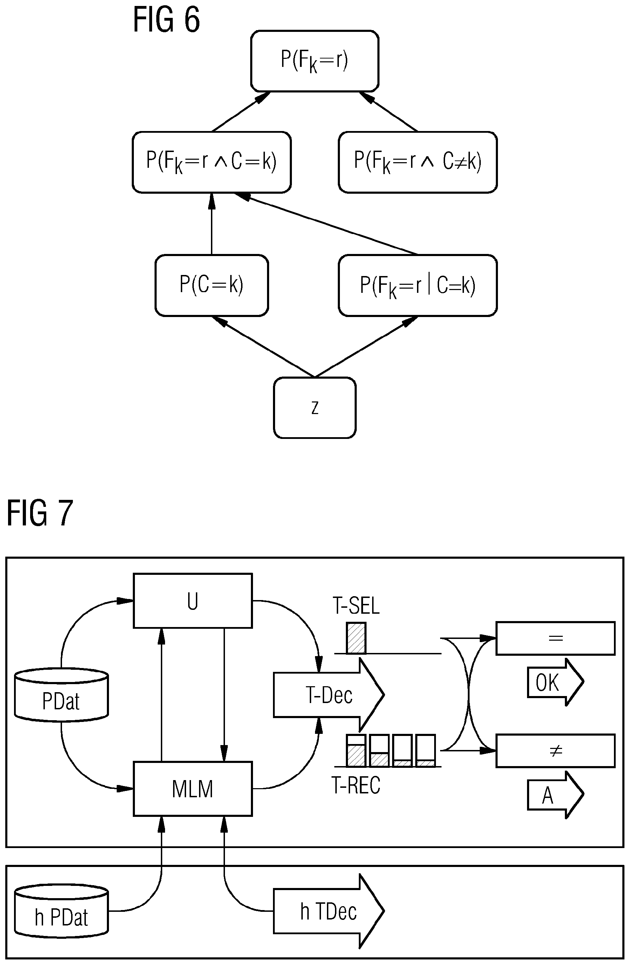

In a possible embodiment, the calculated hidden patient profile vector z can be memorized and stored in a database for further processing. In a possible embodiment, the hidden patient profile vector z of a patient P is automatically recalculated or updated when a new current binary event vector e for the respective patient is received by the clinical decision support system 1. The clinical decision support system 1 can comprise a database or memory for storing the updated hidden patient profile vectors of a group of patients or a plurality of patients. In a possible embodiment, the processing unit 3 is configured to calculate automatically distance metrics between the patient profile vector z of the patient and patient profile vectors z' of other patients stored in the database to quantify similarities between the patient and the remaining patients. The processing unit 3 can provide a list of similar patients ranked according to the calculated distance metrics and output this list via the user interface 9 to the user. The static background information vector m of the patient can be mapped in a possible embodiment by a trained weight mapping matrix to calculate the latent patient information hidden vector p of the patient which is concatenated with the latent medical history hidden vector h for generating the hidden patient profile vector z of the patient. In a possible embodiment, a category probability is computed by the processing unit 3. The category probability indicates whether the recommended therapy does belong to a specific therapy category of a predefined set of different therapy categories C. The processing unit 3 can calculate in a possible embodiment marginal probabilities for therapy recommendations using the hierarchical target regression model HTRM on the basis of the received hidden patient profile vector z and the computed category probability. In a possible embodiment, the therapy recommendations are sorted according to their marginal probabilities to provide a ranking list of possible therapies output to the user via the user interface 9 wherein the user can select a therapy to be applied to the patient from the ranking list. In a possible embodiment, the therapy with the highest marginal probability can be selected automatically and output as the most recommended therapy to the user such as a physician. In a possible embodiment, a deviation alert is automatically generated if the therapy selected by the user differs from the therapy having the highest calculated marginal probability. In a possible embodiment, a machine learning model MLM comprises the hierarchical target regression model HTRM, the RNN encoder 2 and the mapping matrix H. The machine learning model MLM can be trained in a possible embodiment on historical patient data hPDat stored in a database as illustrated in Fig. 7. In a further possible implementation, medical actuators attached to the patient or located in the vicinity of the patient can be automatically controlled in response to the selected therapy.

The clinical decision support system 1 as illustrated in the block diagrams of Figs. 1, 2 can be used in a variety of use cases. In a possible embodiment, the predictive clinical decision support system 1 can be used in a hospital for assisting users in making clinical decisions concerning the therapeutic treatment of a patient. In a possible embodiment, the clinical decision support system 1 as shown in Figs. 1, 2 can be integrated in a data system of a hospital comprising a plurality of terminals connected to each other via a local data network. The user U such as a physician or a nurse can have access to the clinical decision support system 1 via a user interface 9 of a terminal. In a possible embodiment, the processing unit 3 can comprise one or several processors adapted to calculate therapy recommendations for a user which can be displayed to the user via a graphical user interface 9 for selection of a therapy. Accordingly, the user U such as a physician or nurse can select a therapy from a list of therapy recommendations which can be ranked on a displayed list according to calculated marginal probabilities. The user U to whom the rank list of recommended therapy recommendations is displayed can select a suitable therapy for treatment of the patient. For instance, the user U can touch the graphical user interface 9 showing the ranking list of possible therapies and select a specific therapy. In a possible embodiment, the selected therapy is compared with the list of recommended therapies to check whether the selected therapy is the therapy with the highest calculated marginal probability forming the most recommended therapy. In a possible implementation, a deviation alert A or hint is automatically generated by the processing unit 3 if the therapy T-SEL selected or prescribed by the user U such as a physician differs from the recommended therapy T-REC having the highest calculated marginal probability as also illustrated in Fig. 7.

In a possible embodiment, the processing unit 3 of the clinical decision support system 1 is connected to one or more controllers which can control different medical actuators. The medical actuators can be any kind of actuators providing a diagnostic and/or a therapeutic treatment of the patient. Accordingly, the actuators can in a possible implementation activate a medical monitoring device attached to the patient or located in the vicinity of the patient. For instance, the controller connected to the processing unit 3 can activate a blood pressure measurement device in response to the therapy selected by the user. Further, the controller connected to the processing unit 3 of the clinical decision support system 1 can activate an actuator to supply a specific drug into a blood vessel of the patient according to the therapy selected by the user. In a possible embodiment, one or several controllers are connected via a control interface to the processing unit 3.

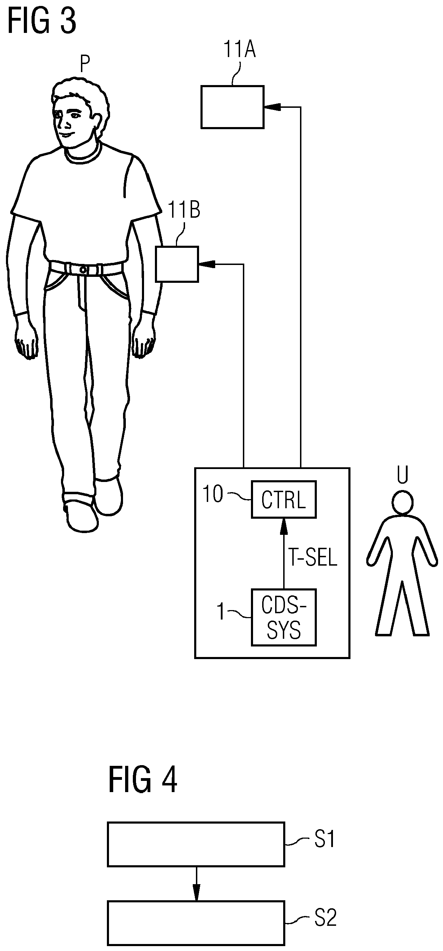

Fig. 3 shows a possible exemplary use case of the predictive clinical decision support system 1 according to an aspect of the present invention. In the illustrated exemplary embodiment, the predictive clinical decision support system 1 is adapted to output the therapy T-SEL selected by the user U such as a physician to a local control unit 10. The local controller 10 can control in the illustrated embodiment a first actuator 11A located in the vicinity of the patient P and a second actuator 11B attached to the patient P. The actuator 11A may for instance activate an observation camera located in the vicinity of the patient P. The other actuator 11B can for instance activate a blood pressure measuring device and/or a drug supply device. Accordingly, in response to the selected therapy T-SEL, one or several actuators 11 performing a diagnostic measurement of patient parameters and/or performing a therapeutic treatment of the patient P can be activated and controlled by the controller 10 in response to the selected therapy T-SEL provided by the predictive clinical decision support system 1.

Fig. 4 shows a flowchart of a possible exemplary embodiment of a method according to an aspect of the present invention. In the illustrated embodiment, the method for providing a predictive clinical decision support comprises two main steps. In a first step S1, a time series of medical events related to a patient P is encoded by a recurrent neural network, RNN, encoder 2 to generate a latent medical history hidden vector h representing the medical history of the patient P. In a second step S2, therapy recommendations T-REC for a user U, such as a physician, are calculated by a processor based on the generated latent medical history hidden vector h using a predefined hierarchical target regression model HTRM stored in a memory. The generated medical history hidden vector h can be extended in a possible implementation with a latent patient information hidden vector p representing the static patient background information of the patient P to generate a hidden patient profile vector z. The generated latent medical history hidden vector h and the latent patient background information hidden vector p can be concatenated in step S2 to provide the patient profile vector z which is supplied to the hierarchical target regression model HTRM for calculating the therapeutic recommendations concerning the patient which are then output to the user via a user interface. In a possible embodiment, in step S2, further distance metrics are calculated between the patient profile vector z and patient profile vectors z' of other patients read from a database to quantify similarities between the patient P and other patients and to provide a list of similar patients ranked according to the calculated distance metrics.

Fig. 5 shows a possible exemplary embodiment of a predictive clinical decision support system 1 according to the present invention. Fig. 5 shows an exemplary implementation of a hierarchical target regression model HTRM having in the illustrated embodiment two layers. As can be seen in Fig. 5, the patient profile vector z is supplied to the hierarchical target regression model HTRM to calculate marginal probabilities. The log linear mapping unit 6 generates from the stored static vector information vector m of the patient P a latent patient information hidden vector p of the patient P. In the illustrated embodiment, a static background information vector m of the patient P is mapped by a trained weight mapping matrix H to calculate a latent patient information hidden vector p of the patient P. The RNN encoder 2 calculates the latent medical history hidden vector h of the patient P from the binary event vectors e at different time steps t as shown in Fig. 5. The advantage of the RNN encoder 2 in handling the medical history of the patient P having variable lengths lies in the fact that the RNN encoder 2 can extract from a sequence of arbitrary length a fixed sized hidden vector h as shown in Fig. 5. Furthermore, with a well-designed advanced RNN encoder 2 such as an LSTM encoder or a GRU-RNN encoder, this vector is capable of representing the whole sequence by learning to remember the relevant medical events and ignoring or suppressing the irrelevant medical events for a specific predictive task. A predictive data model such as the hierarchical target regression model HTRM that consumes the generated latent medical history hidden vector h provides a better performance than predictive models that are based on raw medical features. A further advantage of the encoded vectors, i.e. the generated latent medical history hidden vector h, and the latent patient background information hidden vector p is that they allow to quantify distances and/or similarities between patients with a variable long medical history, by defining distance metrics in a latent vector space. In a possible embodiment, the proposed model provides physicians in a clinic or hospital with recommended therapies and with a list of similar patient cases in order to support these therapeutic recommendations. The list of similar patient cases can comprise patients having received therapies similar to the recommended therapy so that the user or physician U is encouraged to interpret the therapeutic recommendations T-REC with more confidence. Due to the fact that the patients P can have medical histories of variable length, it is nontrivial to apply common distance metrics directly to the patient features to quantify the similarity. For instance, in a conventional system, it is impossible to mathematically calculate the distance between a patient P having undergone a breast conservation surgery and another patient with a mastectomy followed by three successive radiotherapies.

There are two major advantages of using a hierarchical response model to generate predictions. Firstly, the user U such as a physician does not only receive a recommendation of a therapy, e.g. Brachytherapy with a specific probability score, but also a recommendation score of a radiotherapy in the first place. It may happen that, although the Brachytherapy is preferred compared to the other radiotherapy variants, the radiotherapy by itself, however, is not preferable at all. This does give the physician or user U more transparency in the prediction and may avoid unfavorable decisions and misinterpretation of the recommendations given by the system.

The RNN encoder 2 does encode the whole sequence of event features into a single fixed sized vector h. Since the weights W and U are shared among all time steps t, the RNN encoder 2 does not require all sequences to have the same length. Further, the generated latent medical history hidden vector h output by the RNN encoder 2 is extended in a preferred embodiment with another latent vector p representing the static patient background information. The concatenation of both hidden vectors functions as a patient profile vector z in a latent vector space. In other words, similar patients will also be placed closer to each other in this z-vector space and can therefore receive similar predictions, i.e. therapeutic recommendations.

In the hierarchical response model or hierarchical target regression model HTRM, it is possible in a first step to model the probability that each of different main therapy categories is chosen at time step t* for a patient P using for instance a Bernoulli variable C:

$$\mathbb{P}\left(C_i^{[t*]} = k \mid m_i, \{x_i^{[t]}\}_{t=1}^{t*}\right) = \sigma((z_i^{[t*]})^T \gamma^k) \quad,$$

(1)

In the exemplary HTRM shown in Fig. 5 there are three main categories including i.e. radiotherapy (RT), drug therapy (DT) and surgery therapy (ST). Each main category can comprise several therapeutic treatments, i.e. radiotherapeutic treatments rT, drug treatments dT and surgical treatments sT.

Then, in a second step, given a specific therapy category k and given a number of therapy features in this category lk it is possible to model the lk-th multinominal distributed feature variable F as follows:

$$\mathbb{P}\left(F_{i,k,l^k}^{[t*]} = r \mid C_i^{[t*]} = k, m_i, \{x_i^{[t]}\}_{t=1}^{t*}\right)$$

$$= \frac{\exp\{(z_i^{[t*]})^T \beta_{k,l^k,r}\}}{\sum_{\forall r'} \exp\{(z_i^{[t*]})^T \beta_{k,l^k,r'}\}}$$

(2)

wherein k indicates a number of a main category C of treatment,
m is the static background information vector of the patient,
x is the sequential information consisting of the binary event vectors e,
σ is a sigmoid activation function,
z is the patient profile vector,
C is a category,
γ is a weight factor and

β is a weighting or regression coefficient.
If, for example, a therapy category k can suggest surgery, whose features consist of e.g. several Bernoulli variables, this leads to:

$$\mathbb{P}\left(F_{i,k,l^k}^{[t*]} = r \mid C_i^{[t*]} = k, m_i, \{x_i^{[t]}\}_{t=1}^{t*}\right)$$
$$= \sigma\left((z_i^{[t*]})^T \beta_{k,l^k,r}\right) \; ,$$

$$(3)$$

The product does yield a joint probability of both therapy feature and category C as:

$$\mathbb{P}\left(F_{i,k,l^k}^{[t*]} = r \; \wedge \; C_i^{[t*]} = k \; \middle| \; m_i, \{x_i^{[t]}\}_{t=1}^{t*}\right)$$

$$(4)$$

Due to the fact that:

$$\mathbb{P}\left(F_{i,k,l^k}^{[t*]} = r \; \wedge \; C_i^{[t*]} \neq k \; \middle| \; m_i, \{x_i^{[t]}\}_{t=1}^{t*}\right) = 0$$

$$(5)$$

In all cases, this joint probability is equal to the marginal probability:

$$\mathbb{P}\left(F_{i,k,l^k}^{[t*]} = r \; \middle| \; m_i, \{x_i^{[t]}\}_{t=1}^{t*}\right)$$

$$(6)$$

This is also illustrated in Fig. 6 illustrating how recommendations or marginal probabilities are calculated from the input patient profile hidden vector z.

The predictive clinical decision support system 1 according to the present invention can have access to the historical patient data hPDat and can adjust in a possible embodiment itself to mimic the therapeutic decisions T-Dec. The machine learning model MLM encompassing the RNN encoder 2, the mapping matrix H and the hierarchical target regression model HTRM can be trained using the historical patient data hPDat and historical therapy decisions hTDec taken in the past as shown in Fig. 7.

The method and system 1 for providing a predictive clinical decision support to a user can be implemented in a wide range of use cases. The method and system 1 can be implemented as part of a clinical support system of a hospital. Further, the method and system 1 can be implemented in a distributed system having a remote database connected to distributed terminals via a data network such as local data network or the internet. The terminals with the user interface for the user U such as a physician can be connected to the system 1 via a wired or wireless link. The process of the medical events and the calculation of the therapeutic recommendations T-REC can be performed by a central processing unit or by distributed processing entities connected with each other through a network. Further, the hierarchical target regression model HTRM can be configured via a configuration interface for the respective use case. Therapeutic and/or diagnostic and/or monitoring devices 11 can be activated or deactivated automatically in a possible embodiment in response to the calculated therapeutic recommendations issued to the user and selected by the user U for performing the treatment of the patient P. The user U has the possibility to select and/or overrule a recommended therapeutic recommendation calculated by the processing unit 3 of the system 1. The calculated therapeutic recommendations T-REC can be output to one or several users at the same time. In a possible implementation, the selection of the therapy can be performed by several users, e.g. in a voting process. In a possible implementation, a terminal of the user U having the user interface can be a remote terminal connected to the processing unit 3 of the clinical decision support system 1 via a data network. In a still further possible embodiment, the patient data PDat of a plurality of patients treated in different hospitals can be pooled in a central database used by the clinical decision support system 1 for calculating the therapeutic recommendations T-REC.

In a further possible implementation, the encoding in step S1 of the method illustrated in Fig. 4 and the calculation step S2 can be performed by an application program having access to the medical events of the patient P and to the predefined hierarchical target regression model HTRM. In a possible implementation, this clinical decision support application can be executed by an execution engine implemented in a mobile device. In a further possible embodiment, the clinical decision support application can be executed by a processor of a mobile device such as a laptop or a smartphone. In a possible implementation, the calculated therapeutic recommendations T-REC of the system 1 can be benchmarked with therapeutic recommendations of one or several users U such as physicians of different hospitals to compare therapeutic results with each other.

**Claims**

1. A method for providing a predictive clinical decision support comprising the steps of:

   (a) encoding (S1) a time series of medical events related to a patient (P) by a recurrent neural network, RNN, encoder (2) to generate a latent medical history hidden vector, h, representing the medical history of the patient (P); and
   (b) calculating (S2) therapy recommendations (T-REC) for a user (U), in particular a physician or a nurse, by a processor (3) based on the generated latent medical history hidden vector, h, of the patient (P) using a predetermined hierarchical target regression model (HTRM) stored in a memory (8).

2. The method according to claim 1 wherein the generated latent medical history vector, h, is extended with a latent patient information hidden vector, p, representing static patient background information of the patient (P) to generate a hidden patient profile vector, z.

3. The method according to claim 2 wherein the generated latent medical history hidden vector, h, and the latent patient background information hidden vector, p, are concatenated to provide the patient profile vector, z, supplied to the hierarchical target regression model (HTRM) for calculating therapy recommendations (T-REC) concerning the patient (P) output to the user (U) via an interface (9).

4. The method according to any of the preceding claims 1 to 3 wherein distance metrics are calculated between the patient profile vector, z, of the respective patient (P) and patient profile vectors, z', of other patients to quantify similarities between the patient (P) and other patients and to provide a list of similar patients ranked according to the calculated distance metrics.

5. The method according to any of the preceding claims 1 to 4 wherein the time series of medical events comprising binary event vectors, e, representing features of medical events related to the patient (P) is weighted by the recurrent neural network, RNN, encoder (2) to calculate a current latent medical history hidden vector, h, of the patient (P) representing the medical history of the patient (P).

6. The method according to any of the preceding claims 1 to 5 wherein a static background information vector, m, of the patient (P) is mapped by a trained weight mapping matrix, H, to calculate the latent patient information hidden vector, p, of the patient (P).

7. The method according to any of the preceding claims 1 to 6 wherein the hierarchical target regression model, HTRM, comprises several hierarchical levels for calculating conditional probabilities.

8. The method according to any of the preceding claims 1 to 7 wherein a category probability is computed that the recommended therapy does belong to a therapy category of a predefined set of main therapy categories.

9. The method according to claim 8 wherein marginal probabilities for therapy recommendations (T-REC) are calculated using the hierarchical target regression model (HTRM) on the basis of the hidden patient profile vector, z, and the computed category probability.

10. The method according to any of the preceding claims 1 to 9 wherein therapy recommendations (T-REC) are sorted according to their marginal properties to provide a ranking list of possible therapies output to the user (U) to select a therapy (T-SEL) for the patient (P) from the ranking list.

11. The method according to claim 10 wherein the therapy with the highest marginal probability is automatically selected and output as the recommended therapy to the user (U).

12. The method according to any of the preceding claims 1 to 11 wherein a machine learning model (MLM) comprising the hierarchical target regression model (HTRM) the recurrent neural network, RNN, encoder (2) and the mapping matrix, H, is trained on historical patient data (hPDat) stored in a database.

13. The method according to claim 11 wherein a deviation alert, A, is generated if the therapy selected (T-SEL) by the user (U) differs from the therapy (T-REC) having the highest calculated marginal probability recommended to the user (U).

14. The method according to any of the preceding claims 1 to 13 wherein medical actuators (11A, 11B) attached to the patient (P) or located in the vicinity of the patient (P) are automatically controlled in response to the selected therapy (T-SEL).

15. A clinical decision support system (1) comprising:

(a) a recurrent neural network, RNN, encoder (2) adapted to encode a time series of medical events related to a patient (P) to generate a latent medical history hidden vector, h, representing the medical history of the patient (P); and
(b) a processing unit (3) adapted to calculate therapy recommendations (T-REC) for a user (U) based on the latent medical history hidden vector, h, using a predefined hierarchical target regression model (HTRM) stored in a memory (8).

## FIG 1

1

CDS-SYS

2

RNN-ENC

3

PU

## FIG 2

1

CDS-SYS

4

$e_t$
$e_{t-1}$
$e_{t-2}$
$e_{t-n}$

2

RNN ENC

$\vec{h}$

7

CU

$\vec{z}$

3

PU

TR

9

INT

5

m

6

LLM

$\vec{P}$

HTRM

8

## FIG 3

## FIG 4

EP 3 432 173 A1

# FIG 5

## FIG 6

## FIG 7

Europäisches
Patentamt

European
Patent Office

Office européen
des brevets

# EUROPEAN SEARCH REPORT

Application Number

EP 17 18 1838

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YE ET AL: "Cosine similarity measures for intuitionistic fuzzy sets and their applications", MATHEMATICAL AND COMPUTER MODELLING, PERGAMON PRESS, OXFORD, GB, vol. 53, no. 1-2, 1 January 2011 (2011-01-01), pages 91-97, XP027435994, ISSN: 0895-7177, DOI: 10.1016/J.MCM.2010.07.022 [retrieved on 2010-08-03] * Sect. 3; page 92 * * Sect. 5.2; page 96 * | 1-15 | INV. G06F19/00 ADD. G16H50/20 |
| X | US 2012/232930 A1 (SCHMIDT GUENTER [DE] ET AL) 13 September 2012 (2012-09-13) * paragraph [0007] * * paragraph [0038] * * paragraph [0070] * | 1-15 | |
| X | WO 2009/083833 A1 (KONINKL PHILIPS ELECTRONICS NV [NL]; DUTTA PRADYUMNA [US]; ENNETT COLL) 9 July 2009 (2009-07-09) * page 1, line 9 - page 1, line 13 * * claims 1, 2, 25 * | 1-15 | TECHNICAL FIELDS SEARCHED (IPC) G06F |

The present search report has been drawn up for all claims

| Place of search | Date of completion of the search | Examiner |
|---|---|---|
| Munich | 13 February 2018 | Rivera, Pedro V. |

EPO FORM 1503 03.82 (P04C01)

## ANNEX TO THE EUROPEAN SEARCH REPORT
## ON EUROPEAN PATENT APPLICATION NO.

EP 17 18 1838

This annex lists the patent family members relating to the patent documents cited in the above-mentioned European search report.
The members are as contained in the European Patent Office EDP file on
The European Patent Office is in no way liable for these particulars which are merely given for the purpose of information.

13-02-2018

| Patent document cited in search report | Publication date | Patent family member(s) | Publication date |
|---|---|---|---|
| US 2012232930 A1 | 13-09-2012 | US 2012232930 A1<br>US 2017193660 A1 | 13-09-2012<br>06-07-2017 |
| WO 2009083833 A1 | 09-07-2009 | CN 101911078 A<br>EP 2229643 A1<br>US 2010312798 A1<br>WO 2009083833 A1 | 08-12-2010<br>22-09-2010<br>09-12-2010<br>09-07-2009 |

EPO FORM P0459

For more details about this annex : see Official Journal of the European Patent Office, No. 12/82